# EUROPEAN PATENT SPECIFICATION

(11) **EP 3 083 976 B1**
(45) Date of publication and mention of the grant of the patent: **15.07.2020**
(21) Application number: 14821152.7
(22) Date of filing: 18.12.2014
(51) Int. Cl.: C12P 17/18, C12P 19/62

(54) **PROCESS FOR THE PREPARATION OF TIACUMICIN B**
VERFAHREN ZUR HERSTELLUNG VON TIACUMICIN B
PROCÉDÉ POUR LA PRÉPARATION DE TIACUMICINE B

(30) Priority: 20.12.2013 NO 20131718
(43) Date of publication of application: 26.10.2016
(73) Proprietor: Xellia Pharmaceuticals ApS, 2300 Copenhagen S (DK)
(72) Inventor: HANSEN, Espen Fridtjof, N-3050 Lommedalen (NO); KLEMSDAL, Håvard, N-1162 Oslo (NO)
(74) Representative: Onsagers AS
(86) International application number: PCT/EP2014/078552
(87) International publication number: WO 2015/091851

(56) References cited:
- EP-A2- 2 647 643
- WO-A1-02/06203
- WO-A2-97/36996
- WO-A2-2004/014295
- US-A- 4 918 174

## Description

The present invention relates to an improved process for the preparation and purification of Tiacumicin B. Specifically, the invention relates to a purification of Tiacumicin B from a dried fermentation broth, followed by extraction and chromatography techniques. The process according to the invention is simpler than the processes according to the prior art, and can easily be used on a large scale for commercial production.

Tiacumicin B can be produced as disclosed in US4918174 or WO2004014295.

### Summary of the invention

The present invention concerns a process for preparing Tiacumicin B comprising the steps of drying a fermentation broth prior to extraction with methanol.

In particular, the present invention provides a process for the preparation of Tiacumicin B comprising the steps of:
a) fermentation of a Tiacumicin B producing strain;
b) drying the fermentation broth;
c) extraction of the dried material with methanol
wherein the strain of step a) is selected from the group consisting of *Dactylosporangium* and *Actinoplanes.*

The drying step b) according to the present invention may be performed by spray drying or freeze drying.

The solvent used according to the present method is methanol.

The ratio between solvent volume to dried fermentation broth mass according to the present method may be 1-6 ml/g. According to one embodiment of the present invention, the ratio between solvent volume to dried fermentation broth mass is 2-4 ml/g.

According to the present invention, the Tiacumicin B producing strain to be used in the fermentation step is selected from the group consisting of Dactylosporangium and Actinoplanes.

The various aspects and more of the present invention, including various embodiments, will be described in further detail, with reference to the detailed description, examples and appended drawings.

### Figures:

Figure 1 shows the extraction efficiency at different solvent concentration of a spray dried fermentate.
Figure 2 shows the extraction yield at different methanol concentration of a spray dried fermentate.
Figure 3 shows the yield when extracting a spray dried fermentate at different volumes of 75% v/v aqueous methanol.

### Detailed description:

According to the present invention, any Tiacumicin B producing bacterial strain selected from the group consisting of *Dactylosporangium* and *Actinoplanes* may be used to provide the fermentation broth according to the present invention. According to one embodiment, Tiacumicin B can be produced by fermentation of *Dactylosporangium aurantiacum* subspecies hamdenensis NRRL 18085 or *Actinoplanes deccanensis* ATCC 21983.

The fermentation broth is the liquid nutrient medium comprising the bacterial biomass and the Tiacumicin compounds.

The drying of the fermentation broth can be performed by freeze drying. A preferred freeze drying process includes reducing the temperature to -40°C and the pressure to 200 mTorr, then performing a primary drying at a temperature in the range -5°C to 5°C and thereafter secondary drying at a temperature of about 20°C.

The preferred drying process for the fermentation broth according to the present invention is spray drying. Despite the high temperature in the spray drying process, Tiacumicin B can be recovered in high yield.

A preferred spray drying process includes an in-let temperature of 180-220 °C and an out-let temperature of 80-100 °C. The drying is performed to achieve a water content of 0%-12% w/w, more preferred is a water content of less than 10% w/w; e.g. 2%-8% w/w.

The dried fermentation broth is extracted with methanol.

The preferred pH during extraction is about 5-7, preferably 6-7, more preferably 7. The preferred temperature during extraction is 20-25°C.

The methanol concentration in the extraction solution is subsequently adjusted by evaporation or dilution to a concentration suitable for binding of tiacumicin B on an adsorption resin, e.g. 50% methanol in water. After binding on an adsorption resin, the resin is washed with an aqueous solvent solution to remove unbound impurities, e.g. 50% organic solvent. The bound tiacumicin B can be eluted with a solvent comprising 60-100% organic solvent and 0-40 v/v water.

### Experimental data:

### Example 1

*Dactylosporangium aurantiacum* subspecies hamdenensis NRRL 18085 fermentate was spray dried directly using a Niro Mobile Minor spray dryer with a 2-fluid nozzle. The air pressure was 1.2 bar. Inlet-temperature was ca. 200°C and outlet temperature was 90-92°C. The feeding rate was 0.5-1.0 liters per hour.

The spray dried powder was divided into eight 5ml volumetric flasks with ca. 1 g in each. Solvent was then added up to the 5ml mark. Different solvents were used: ethanol, methanol, isopropanol, n-propanol, acetonitrile, acetone, dimethyl sulfoxide, water. The flasks were shaken in an overhead stirrer for one hour. The slurry was withdrawn and centrifuged. The resulting supernatant was analyzed by HPLC. DMSO was the most efficient solvent in the extraction. However, DMSO is not suitable for production scale due to e.g. high boiling point. Surprisingly, methanol gave highest yield of the other solvents. The yield was 50-100 % higher compared to ethanol, isopropanol, n-propanol, acetonitrile and acetone.

### Example 2

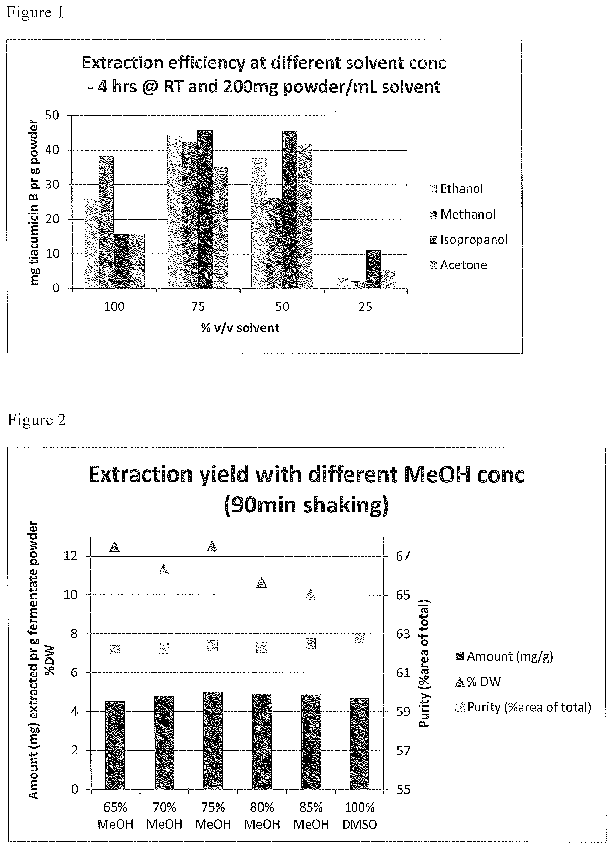

Spray dried *Daclylosporangium aurantiacum* subspecies hamdenensis NRRL 18085 fermentate was extracted with different solvents and different solvent/water compositions. Ethanol, methanol, isopropanol and acetone were used with concentrations of 100%, 75%, 50% and 25% in water. Five times the weight of the dry fermentate was used in volume (i.e. 200 mg powder pr. ml solution). The slurries were shaken in an overhead shaker for four hours. The slurry was centrifuged, and the supernatant was analyzed by HPLC. The results are shown in figure 1.

### Example 3

Spray dried *Dactylosporangium aurantiacum* subspecies hamdenensis NRRL 18085 fermentate was extracted with methanol of different concentrations in water. 1 g fermentate was transferred to a 5ml measuring flask, and methanol/water solution was added to the mark. Concentrations of 65%, 70%, 75%, 80% and 85% methanol in purified water (RO) were tested. The slurries were shaken in an overhead shaker for 1.5 hours. The slurries were centrifuged, and the supernatants were analyzed by HPLC. The dry weights of the supernatants were also determined.

The results are shown in figure 2.

There was a peak in the extraction yield at 75-80% methanol. The dry weight decreased with increasing methanol concentration. The HPLC purity remained the same with all methanol concentrations.

### Example 4

Spray dried *Dactylosporangium aurantiacum* subspecies hamdenensis NRRL 18085 fermentatewas extracted with different volumes of 75% methanol/water. 1 g of fermentate was added 2, 3, 4, 5, and 10 ml 75% methanol in different tubes. The slurries were shaken in an overhead shaker for 1.5 hours. The slurries were centrifuged, and the supernatants were analyzed by HPLC.

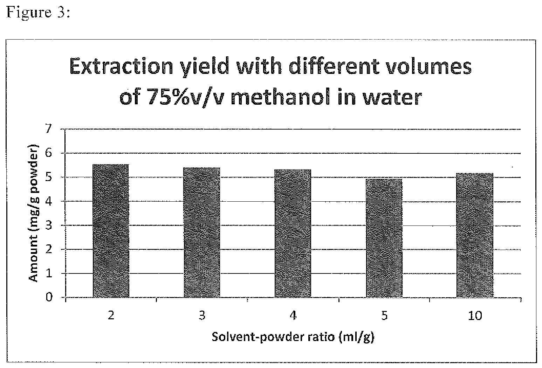

The results are shown in figure 3. The yield was similar for all volumes.

### Example 5

Spray dried *Dactylosporangium aurantiacum* subspecies hamdenensis NRRL 18085 fermentatewas extracted with 75% methanol/water (3 times the weight in volume) under stirring for two hours. The slurry was centrifuged, and concentrated two times in a rotary evaporator to give 50% methanol in the solution. The concentrate was loaded directly on a HP20 resin packed in a column. The column was washed with 50% methanol and eluted with a gradient from 50% to 100% methanol. The yield was 92%.

### Example 6

The drying step of the present invention may be performed by freeze drying both in small and large scale as shown in the below examples.

### 6.1 Small scale freeze drying

4 L *Dactylosporangium aurantiacum* subspecies hamdenensis NRRL 18085 fermentate was poured into to steel trays (height of liquid 1.5-2.0 cm). The trays were mounted in a Virtis Genesis 12ES freeze dryer and frozen to -40°C. After reaching the mentioned temperature they were let standing for two hours. Then the condenser was cooled to ca -50°C and a vacuum of 200 mTorr was established. The shelf temperature was adjusted to -5°C during 600 min. Then the shelf temperature was quickly adjusted to 0°C and kept for 1250 min. Another quick adjustment of the shelf temperature to +5°C was performed, and the product was kept at this temperature for 600 min. The secondary drying was performed by quick adjustment to +20°C upon which the product was left for 1250 min at vacuum (200mTorr). Then the tanks were removed from the freeze dryer and 179 g dry material was obtained.

### 6.2 Large scale freeze drying

Ca 110 L *Dactylosporangium aurantiacum* subspecies hamdenensis NRRL 18085 fermentate was poured into 48 steel trays (height of liquid 1.5-2.0 cm). The trays were placed in two freeze dryers, and frozen to -20°C. After reaching the mentioned temperature, the fermentate was left standing for a while. The condenser was cooled to ca -40°C. Then a vacuum of ca 200 mTorr was established. The shelf temperature was adjusted to +40°C during ca 44 hours. Then the trays were removed from the freeze dryer and 6.6 kg dry material was obtained.

400 g of freeze dried *Dactylosporangium aurantiacum* subspecies hamdenensis NRRL 18085 fermentate was added 2000 ml 80% v/v methanol-water and stirred for 1 hour. Then the mixture was centrifuged at 4500rpm for 15min. The supernatant was decanted (volume of 1550 ml). The sediment was added 1400 ml 80 % v/v methanol-water. The mixture was left over night and stirred for 1 hour the next day. Then it was centrifuged at 4500 rpm for 15min. The supernatant was decanted (volume of 1380ml). Both supernatants were combined to a total volume of 2930 ml. Total yield 79%.

The solution was then evaporated under reduced pressure on a 40°C water bath. The final volume was 1220 ml comprising 40% water as measured by Karl Fisher titration. This solution was loaded on a column packed with a HP20 resin.

## Claims

1. A process for the preparation of Tiacumicin B comprising the steps of:
a) fermentation of a Tiacumicin B producing strain;
b) drying the fermentation broth;
c) extraction of the dried material with methanol,
wherein the strain of step a) is selected from the group consisting of *Dactylosporangium* and *Actinoplanes.*

2. A process according to claim 1, wherein the broth is spray dried or freeze dried.

3. A process according to claim 1, wherein the broth is spray dried.

4. A process according to claim 1, wherein the broth is freeze dried.

5. A process according to any of the above claims, further comprising a chromatography step involving loading of the extraction solution on a column comprising a hydrophobic adsorbent resin, optionally washing before elution.

## Patentansprüche

1. Verfahren zur Herstellung von Tiacumicin B, umfassend die Schritte:
a) Fermentation eines Tiacumicin B erzeugenden Stammes;
b) Trocknen der Fermentationsbrühe;
c) Extraktion des getrockneten Materials mit Methanol, wobei der Stamm von Schritt a) ausgewählt ist aus der Gruppe bestehend aus *Dactylosporangium* und *Actinoplanes.*

2. Verfahren nach Anspruch 1, wobei die Brühe sprüh- oder gefriergetrocknet wird.

3. Verfahren nach Anspruch 1, wobei die Brühe sprühgetrocknet wird.

4. Verfahren nach Anspruch 1, wobei die Brühe gefriergetrocknet wird.

5. Verfahren nach einem der obigen Ansprüche, das ferner einen Chromatographieschritt umfasst, der das Beladen einer Säule, die ein hydrophobes Adsorptionsharz umfasst, mit der Extraktionslösung, gegebenenfalls Waschen vor der Elution, umfasst.

## Revendications

1. Procédé de préparation de Tiacumicine B comprenant les étapes de :
a) fermentation d'une souche produisant de la Tiacumicine B ;
b) séchage du bouillon de fermentation ;
c) extraction du matériau séché avec du méthanol,
dans lequel la souche de l'étape a) est choisie dans le groupe constitué de *Dactylosporangium* et *Actinoplanes.*

2. Procédé selon la revendication 1, dans lequel le bouillon est séché par pulvérisation ou séché par lyophilisation.

3. Procédé selon la revendication 1, dans lequel le bouillon est séché par pulvérisation.

4. Procédé selon la revendication 1, dans lequel le bouillon est séché par lyophilisation.

5. Procédé selon l'une quelconque des revendications précédentes, comprenant en outre une étape de chromatographie comportant le chargement de la solution d'extraction sur une colonne comprenant une résine adsorbante hydrophobe, éventuellement en lavant avant l'élution.
